# EUROPEAN PATENT APPLICATION

(11) **EP 0 046 628 A2**
(43) Date of publication of application: **03.03.1982**
(21) Application number: 81302655.6
(22) Date of filing: 15.06.1981
(51) Int. Cl.: C07F 15/04, B01J 31/28, C07C 2/08

(54) **Process for the preparation of nickel ylides containing directly sulfonated ylide ligands**

(30) Priority: 18.08.1980 US 179078
(71) Applicant: GULF RESEARCH & DEVELOPMENT COMPANY, Pittsburgh Pennsylvania 15230 (US)
(72) Inventor: Beach, David L., Gibsonia Pennsylvania 15044 (US); Harrison, James J., Glenshaw Pennsylvania 15116 (US)
(74) Representative: Huskisson, Frank Mackie

(57) **Abstract**

A process is provided for preparing nickel ylides which are themselves novel compounds defined by the following Formula I 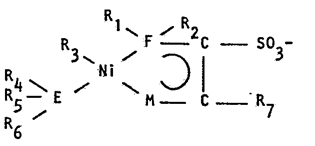 wherein Rᵢ, R₂, R₃, and R₇ are either alike or different members selected from the group consisting of hydrogen, alkyl radicals having from about one to about 24 carbon atoms, preferably from about one to about 10 carbon atoms; aryl radicals having from about six to about 20 carbon atoms, preferably from about six to about 10 carbon atoms; alkenyl radicals having from about two to about 30 carbons atoms, preferably from about two to about 20 carbon atoms; cycloalkyl radicals having from about three to about 40 carbon atoms, preferably from about three to about 30 carbon atoms; aralkyl and alkaryl radicals having from about six to about 40 carbon atoms, preferably from about six to about 30 carbon atoms; a halogen radical selected from the group consisting of fluorine, chlorine, bromine and iodine, preferably chlorine; a hydroxyl group; an alkoxy or aryloxy group; and a hydrocarbyl group, such as defined above, carrying halogen, hydroxyl or alkoxy or aryloxy; R₄, R₅ and R₆ are either alike or different members selected from the group consisting of a hydrocarbyl group as defined above, a sulfonato group (-SO₃⁻) or a sulfonated hydrocarbyl group; M is sulfur or oxygen, preferably oxygen; E is phosphorus, arsenic, antimony or nitrogen, preferably phosphorus; and F is phosphorus, arsenic or antimony, preferably phosphorus. The process comprises sulfonating a metal ylide defined by the following Formula II: 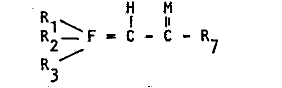 to obtain the sulfonated ylide defined by the following Formula III: 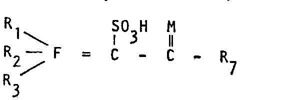 wherein R₁, R₂, R₃, R₇, F and M are as defined above. The sulfonated ylide is reacted with a base to obtain the metal ylide defined by the following Formula IV: 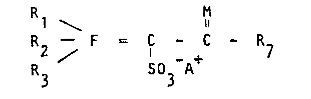 wherein R₁, R₂, R₃, R₇, F and M are as defined above and A is the cationic portion of the base used. This ylide is then reacted with (1) a zero valent nickel compound and (2) a ligand having the formula: 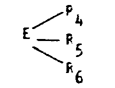 wherein R₄, R₅, R₆ and E are as defined above.

## Description

Reference is made to applicants' following U.S. applications:
U.S. Patent application Serial No.179079, filed 18 Auqust 1980, entitled "Nickel Ylides".
U.S. Patent application Serial No. 179075, filed 18 August 1980, entitled "Process for the Preparation of Nickel Ylides Containing Sulfonated Group V Ligands".
U.S. Patent application Serial No. 197080, filed 18 August 1980, entitled "Process for the Preparation of Nickel Ylides Containing Ylide Ligands with a Sulfonated Group V Component".
U.S. Patent application Serial No. 179076, filed 18 August 1980, entitled "Process for the Oligomerization of Ethylene"
U.S. Patent application Serial No. 179005, filed 18 August 1980, entitled "Process for the Oligomerization of Ethylene in Methanol."

The disclosures of the foregoing applications are hereby incorporated by reference.

### Field Of The Invention

The present invention relates to a novel process for preparing nickel ylides which are useful as catalysts for the oligomerization of ethylene. This invention also relates to novel intermediates obtained in such a process and to the methods for preparing such intermediates.

### Description Of The Prior Art

It is well known in the art to use a variety of catalysts to oligomerize ethylene to higher molecular weight olefins. The term "oligomerize" has been employed, and is employed herein to describe the conversion of lower olefins such as ethylene to olefinic products of higher molecular weight, e.g., to dimer, trimer, tetramer and the like. The reaction rate and product distribution obtained are highly dependent on the exact catalyst composition and the reaction conditions employed. Two such general classes of catalysts are the "Ziegler" types consisting of aluminum trialkyls and the "Ziegler-Natta" types consisting of aluminum alkyls or alkyl halides and titanium halides. Major disadvantages of aluminum alkyl catalysts are their highly reactive and pyrophoric nature and the fact that they must be used at relatively high temperatures, e.g., 200-275^{*}C. and pressures, e.g., 2000-4000 psig (13,790 to 27,580 kPa). Although much milder reaction conditions are used when the aluminum alkyls are used in conjunction with titanium halides, product quality and ease of catalyst separation from products of both of these prior art types of catalysts are not as high as desired.

An article by W. Keim, F.H. Kowaldt, R. Goddard and C. Kruger entitled "Novel Coordination of (Benzoyl- methylene)triphenylphosphorane in a Nickel Oligomerization Catalyst", in Angew. Chem. Int. Ed. Engl. (1978) No. 6, page 466, discloses the preparation of a nickel ylide by the following reaction: 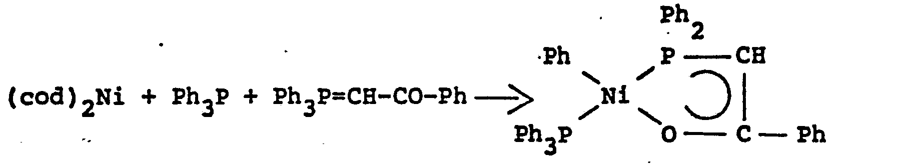 wherein "cod" represents 1,5-cyclooctadiene and "Ph" represents phenyl. It is reported that the resultant nickel ylide converts ethylene into alpha olefins or polyethylene.

### Summary Of The Invention

A novel process has now been found for preparing nickel ylides which are themselves novel compounds defined by the following Formula I: 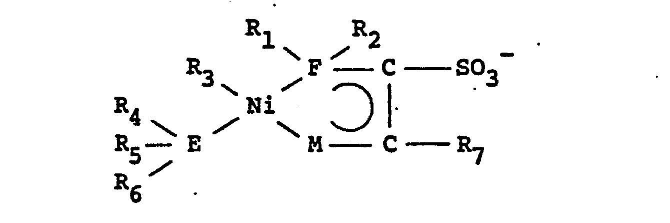 wherein Rₗ, R₂, R₃, and R₇ are either alike or different members selected from the group consisting of hydrogen, alkyl radicals having from about one to about 24 carbon atoms, preferably from about one to about 10 carbon atoms; aryl radicals having from about six to about 20 carbon atoms, preferably from about six to about 10 carbon atoms; alkenyl radicals having from about two to about 30 carbons atoms, preferably from about two to about 20 carbon atoms; cycloalkyl radicals having from about three to about 40 carbon atoms, preferably from about three to about 30 carbon atoms; aralkyl and alkaryl radicals having from about six to about 40 carbon atoms, preferably from about six to about 30 carbon atoms; a halogen radical selected from the group consisting of fluorine, chlorine, bromine and iodine, preferably chlorine; a hydroxyl group; an alkoxy or aryloxy group; and a hydrocarbyl group, such as defined above, carrying halogen, hydroxyl or alkoxy or aryloxy; R₄, R₅ and R₆ are either alike or different members selected from the group consisting of a hydrocarbyl group as defined above, a sulfonato group (-SO₃⁻) or a sulfonated hydrocarbyl group; M is sulfur or oxygen, preferably oxygen; E is phosphorus, arsenic, antimony or nitrogen, preferably phosphorus; and F is phosphorus, arsenic or antimony, preferably phosphorus. The process comprises sulfonating a metal ylide defined by the following Formula II: 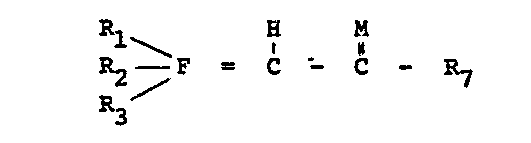 to obtain the sulfonated ylide defined by the following Formula III: 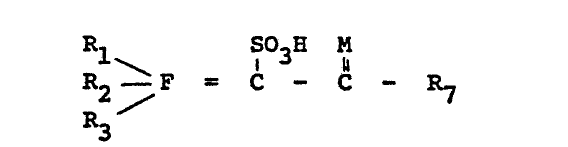 wherein R₁, R₂, R₃₁ R₇, F and M are as defined above. The sulfonated ylide is reacted with a base to obtain the metal ylide defined by the following Formula IV: 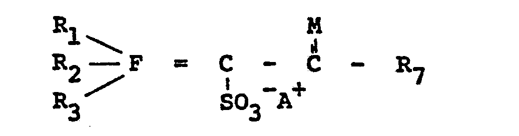 wherein R₁, R₂, R₃, R₇, F and M are as defined above and A is the cationic portion of the base used. This ylide is then reacted with (1) a zero valent nickel compound and (2) a ligand having the formula: 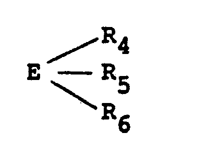 wherein R₄, R₅, R₆ and E are as defined above.

The presence of the sulfonato group in the nickel ylides obtained in the process of this invention induces solubility in polar solvents such as water or methanol. This facilitates product removal and separation from the reaction media or the use of extractive techniques, e.g., by the use of aqueous ammonium hydroxide, not possible with the corresponding nickel ylides which do not contain a sulfonato group.

### Description Of The Preferred Embodiments

In the process of this invention, the first step involves sulfonating a metal ylide defined by the following Formula II: 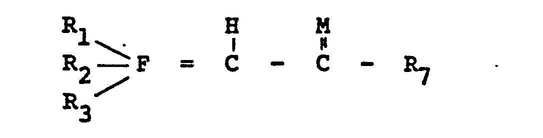 wherein each of R₁, R₂, R₃, R₇₁ F and M is as defined above to obtain the following sulfonated ylide defined by the following Formula III. 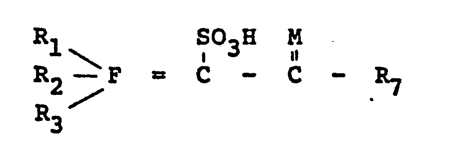 wherein each of R₁, R₂, R₃, R₇, F and M is as defined above. In some cases, for example, where R₁, R₂, R₃ and R₇ are phenyl, M is oxygen and F is phosphorous, the following following Formula IIIa may more accurately describe the structure:

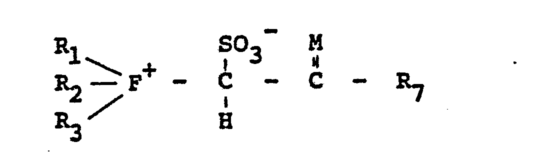

This first step can be done, for example, by dissolving the ylide of Formula II in a suitable solvent, for example, a halogenated hydrocarbon, such as chloroform, dichloroethane, methylene chloride or methyl chloroform, or a hydrocarbon solvent, such as heptane or hexane and then adding S0₃ to the resulting solution. The ylide and sulfonating agent are generally employed in equal molar amounts, although excess sulfonating agent can be present, if desired. Temperatures can be in the range of about 0° to about 200°C., preferably from about 20° to about 100°C., pressures can be elevated, although atmospheric pressure is preferred, and reaction times can vary from about five minutes to about 24 hours, preferably from about ten minutes to about four hours.

At the end of the reaction time the compounds defined by Formula III or IIIa are recovered by any suitable means. If the sulfonated desired product is solid, recovery can be effected by filtration, decantation or by centrifuging. If the desired product is dissolved in the reaction medium, recovery can be effected by distillation to remove the solvent therefrom.

The sulfonated product is converted to the corresponding ylide by reacting the same with a base, such as an alkali metal hydroxide (sodium or potassium hydroxide), an alkyl or aryl lithium (n-butyl lithium, methyl lithium or phenyl lithium), an alkoxide (sodium methoxide or potassium t-butoxide), a hydrocarbyl-substituted ammonium hydroxide (benzyltrimethylammonium hydroxide), ammonium hydroxide, ammonia, etc, to produce the following novel ylide defined by Formula IV: 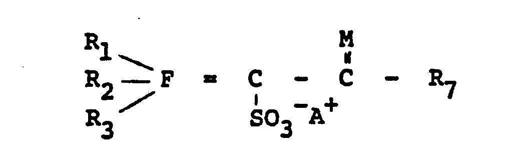 wherein R₁, R₂, R₃, R₇, F, M and A are as defined above. This can be done, for example, by suspending or dissolving the sulfonated ylide in a suitable liquid, such as water, an alcohol (ethanol or isopropanol), an aromatic (benzene or toluene), a hydrocarbon (hexane or heptane), etc. The reaction temperature can range from about room temperature to about 200°C., preferably from about room temperature to about 50°C., and the reaction time from about one minute to about four hours, or even longer, but preferably from about one to about two hours. Elevated pressures can be used, although atmospheric pressure will suffice. If the ylide obtained is a solid, recovery can be effected by filtration, decantation or by centrifuging. If the ylide is dissolved in the solvent, simple distillation is sufficient to remove the solvent, leaving behind the solid ylide.

The sulfonated ylide defined by Formula IV is reacted with (1) a ligand defined by the formula: 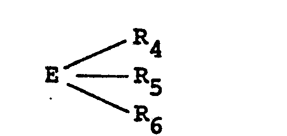 wherein R₄, R₅, R₆ and E are as defined above, and (2) a zero valent nickel compound. Specific examples of ligands that can be used include: allyldiphenylphosphine; benzyldi- phenylphosphine; bis(3-aminopropyl)-phenylphosphine; bis(2-cyanoethyl)phenylphosphine; bis(m-fluorophenyl)phosphinous chloride; 4-bromophenyldiphenylphosphine; n-butyldiphenylphosphine; t-butyldiphenylphosphine; 2-cyanoethyldiphenyl- phosphine; cyclohexyldiphenylphosphine; n-decylphenylphos- phine; diallylphenylphosphine; di-n-amylphenylphosphine; di-sec-butylphenylphosphine; di-cyclohexylphenylphosphine; diethylphenylphosphine; di-n-heptylphenylphosphine; di-n-hexylphenylphosphine; dimethylphenylphosphine; dimethyl- p-tolylphosphine; diphenyl-n-butoxyphosphine; diphenyl- chlorophosphine; diphenylenephenylphosphine; diphenyl- ethoxyphosphine; diphenylmethoxyphosphine; diphenylphosphine; beta-diphenylphosphinoethyltriethoxysilane; di-iso- propylphenylphosphine; di-o-tolylphenylphosphine; divinyl- phenylphosphine; ethyldiphenylphosphine; n-hexyldiphenylphosphine; o-methoxyphenyldiphenylphosphine; (2-methylbutyl)diphenylphosphine; methyldiphenylphosphine; methyl- ethylphenylphosphine; methylphenylphosphine; neomenthyldi- phenylphosphine; pentafluorophenyldiphenylphosphine; (2-phenylbutyl)diphenylphosphine; phenyldi-n-butoxyphosphine; phenyldichlorophosphine; phenyldiethoxyphosphine; phenyl- dimethoxyphosphine; phenylphosphine; isopropyldiphenyl- phosphine; n-propyldiphenylphosphine; o-tolyldiphenylphosphine; p-tolyldiphenylphosphine; tribenzylphosphine; tris-(m-chlorophenyl)phosphine; tris(p-chlorophenyl)phosphine; tri(I-naphthyl)phosphine; triphenylphosphine; tris(4-dimethylaminophenyl)phosphine; tris(p-fluorophenyl)phosphine; tris(o-methoxyphenyl)phosphine; tris(p-methoxyphenyl)phosphine; tri-o-tolylphosphine; tri-m-tolylphosphine; tri-p-tolylphosphine; vinyldiphenylphosphine; sodium diphenylphosphinebenzene-3-sulfonate; disodium phenylphosphine-bis(benzene-3-sulfonate); dimethylphenyl- arsine; methyldiphenylarsine; triphenylarsine; tri-p-- tolylarsine; diphenylchloroarsine; triphenylantimony; triphenylamine; tribenzylamine; methyldiphenylamine; di- methylphenylamine; bis(2-cyanoethyl)phosphine; bis(di- methylamino)methylphosphine; t-butyldichlorophosphine; 2-cyanoethylphosphine; cyclohexylphosphine; di-t-butyl- chlorophosphine; dicyclohexylphosphine; diethylethoxyphos- phine; d iethyl-iso-propoxyphosphine; diethylphosphine; triallylphosphine; tri-iso-butylphosphine; tri-n-butylphosphine; tri-sec-butylphosphine; tri-t-butylphosphine; triethylphosphine; tri-n-hexylphosphine; trimethylphosphine; trifluorophosphine; tri-iso-propylphosphine; tri-n-propylphosphine; tris(2-cyanoethyl)phosphine; tris(di- methylamino)phosphine; tris(trimethylsilyl)phosphine; tri-n-butylantimony; triethylarsine; trimethylarsine; methyl- diiodoarsine; trimethylamine; triethylamine; tributylamine; tripropylamine; dimethylamine; di-n-hexylamine; dicyclohexylamine; diethylamine; tricyclohexylamine; ammonia; and phosphine. Specific examples of zero valent nickel compounds which can be used include: tris(triphenylphosphine)nickel;. bis(cyclooctadiene)nickel; tetra- kis(triphenylphosphine)nickel; bis(norbornadiene)nickel; (cycloocta-ly5-diene)duroquinone nickel; (dicyclopentadiene)duroquinone nickel; bis(tetracyclone)nickel; tetra- kis(triethylphosphine)nickel; tris(triethylphosphine)-nickel; bis(triphenylphosphine)nickel dicarbonyl; nickel carbonyl; nickel(II)acetylacetonate; nickelocene; bis(triethylphosphine)nickel(II)chloride; tetrakis(trifluorophosphine)nickel; nickel acetate; nickel bromide; nickel car bonate; nickel chloride; nickel fluoride; nickel iodide; nickel nitrate; nickel sulfate; nickel 2,4-pentanedionate; bis π -allyl nickel; and nickel dichloride hexaamine. ,

In this step, approximately equal molar amounts of each of the three reactants defined above are dissolved in any suitable unreactive solvent, such as toluene, tetrahydrofuran, dioxane, or other unreactive hydrocarbon solvents, and stirred while maintaining a temperature of about 0° to about 100°C., preferably room temperature, for about one-half hour to about 48 hours, preferably about three to about 20 hours, sufficient to ensure complete reaction. Any suitable pressure can be used, although atmospheric pressure is preferred. The solvent can be removed from the reaction mixture in any suitable manner, for example, by distillation, including vacuum distillation, if necessary, leaving behind the novel compound defined above. On the other hand, a second solvent in which the desired product is insoluble, such as heptane, can be added to the reaction product to precipitate the novel compound therein. The novel compound can be recovered, for example, by filtration, decantation or by centrifuging.

Specific examples of nickel ylides which can be prepared by the practice of this invention are set forth in Table I. In this table and as used elsewhere herein, "Ph" represents phenyl and "Et" represents ethyl.

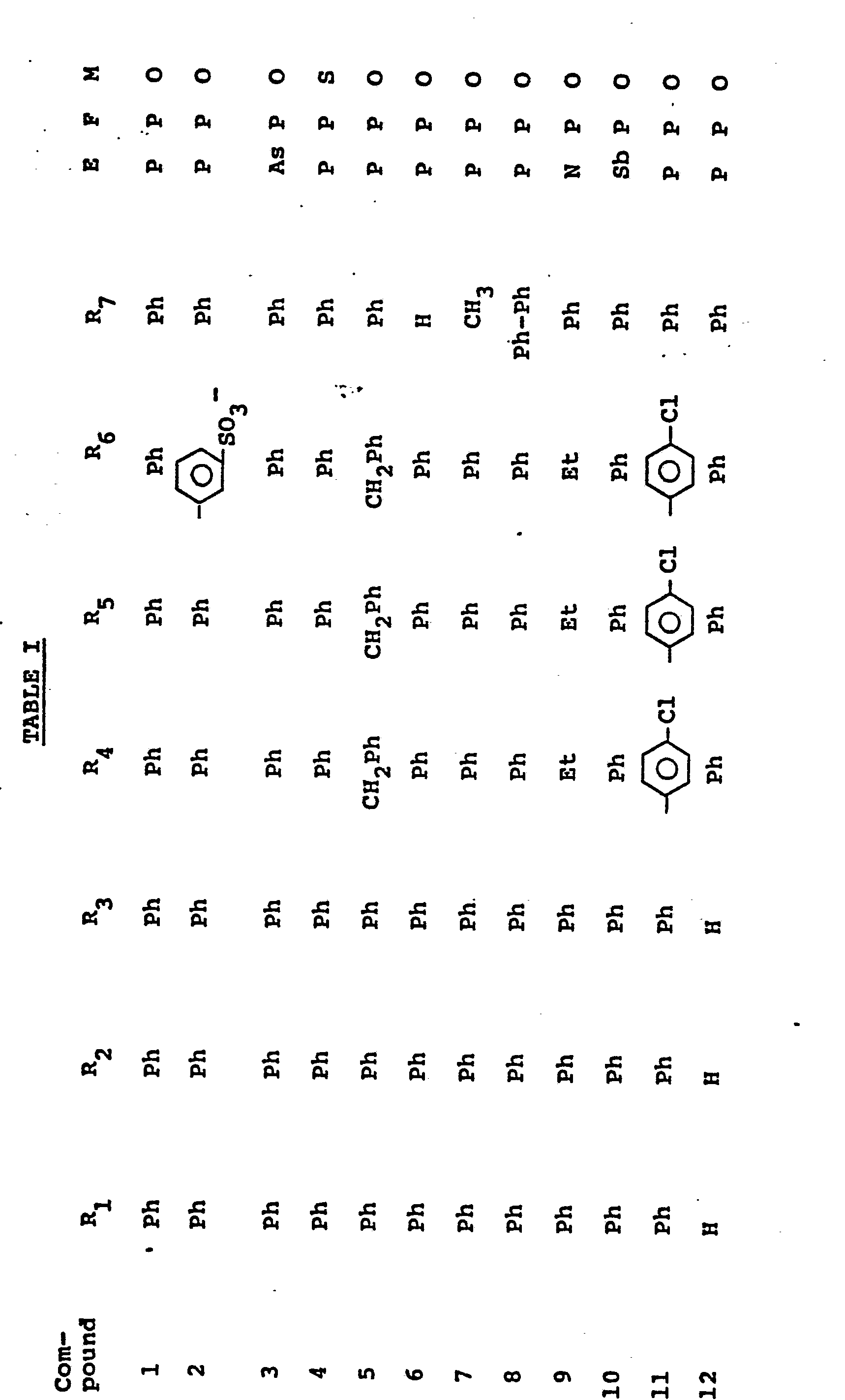

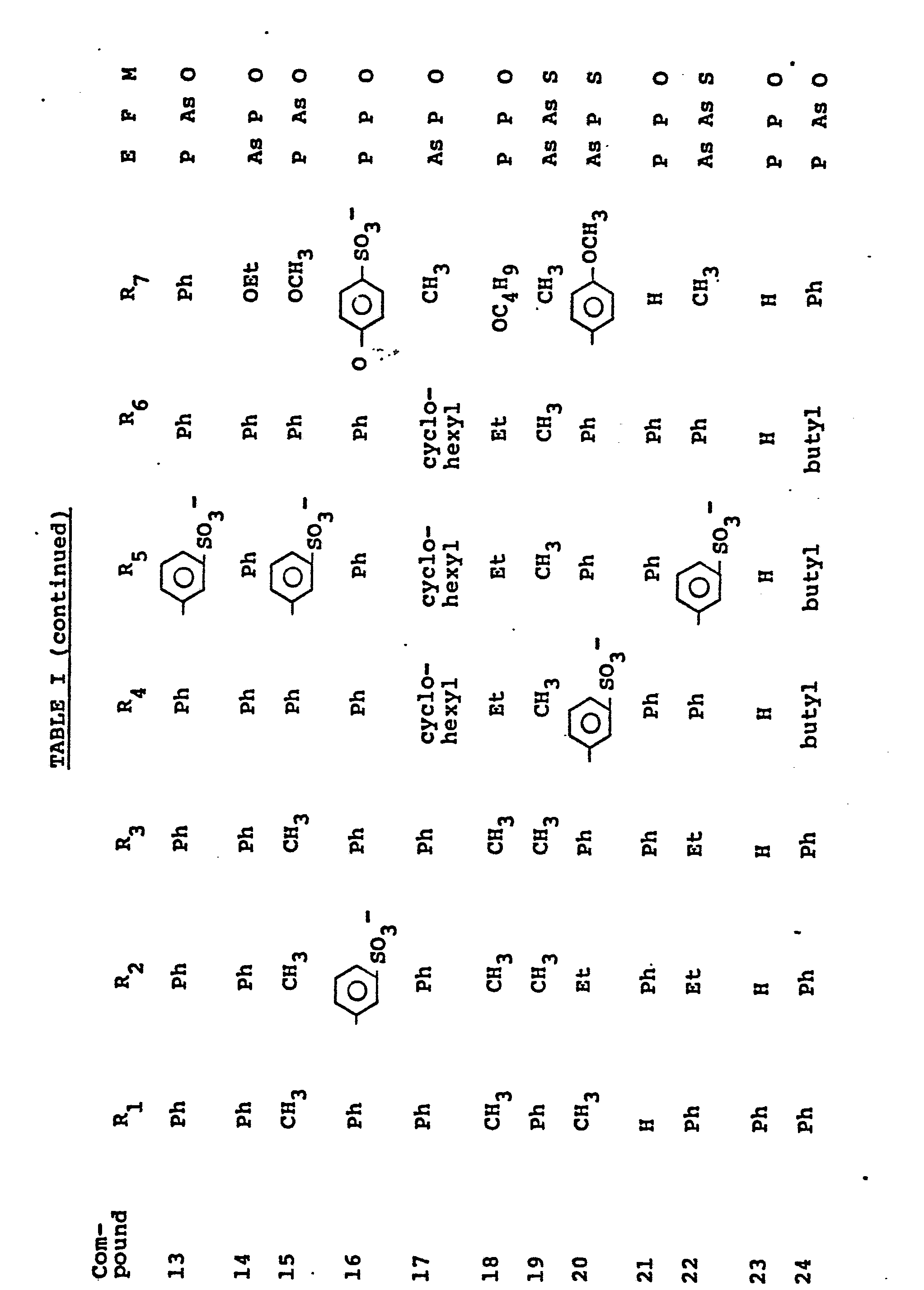

The following examples illustrate the invention, and are not intended to limit the invention, but rather, are presented for purposes of illustration. Example I illustrates the preparation of nickel ylides in accordance with the practice of this invention; and Examples II and III illustrate the use of a nickel ylide to oligomerize ethylene.

### Example I

To 4.01 grams of pyridine (0.05 mole) in 250 milliliters of dichloroethane there was added 6.97 grams of sulfur trioxide (0.087 mole) at 0°C. under nitrogen. After stirring for 0.5 hour, a solution of 19.05 grams of unsubstituted benzoylmethylenetriphenylphosphorane (0.05 mole) in 200 milliliters of dichloroethane was added. This was then heated to reflux for one hour. The reaction mixture was cooled to room temperature and the solvent removed in vacuo. The resulting product was then suspended in ethyl alcohol and filtered to give 19.7 grams of a white solid of the following phosphonium salt in 86 percent yield:

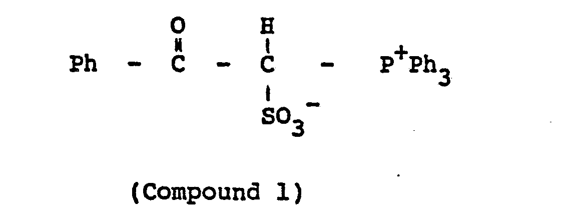

Compound 1 was also prepared as follows.- To 29 grams of benzoylmethylenetriphenylphosphorane (0.076 mole) in 500 milliliters of dichloroethane at 25°C. under nitrogen there was added 5.47 milliliters of sulfur trioxide (0.132 mole). After stirring for 18 hours the solvent was removed in vacuo. Then 450 milliliters of ethanol and 50 milliliters of water were added and the mixture stirred for one-half hour. The product was filtered and washed with ether to give 31.8 grams, 87 percent yield, of Compound 1.

Compound 1 was then suspended in water and titrated with 10 percent aqueous sodium hydroxide to a phenolphthalein end point. The water was then removed in vacuo and final traces removed via ethanol azeotrope to give 20.7 grams of the following novel ylide in 86 percent yield:

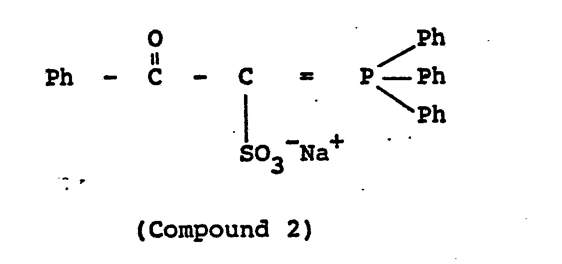

The novel nickel ylide, defined below as Compound 3, was prepared as follows. To 1.38 grams of bis-(cyclooctadiene)nickel (five millimoles) in 30 milliliters of tetrahydrofuran there was added a mixture of 1.31 grams of triphenylphosphine (five millimoles) and 2.41 grams of Compound 8 (five millimoles) dissolved in 70 milliliters of tetrahydrofuran. The reaction mixture was stirred for 18 hours at room temperature, after which solvent was removed in vacuo. The resulting solid was dissolved in toluene and filtered. A yellow solid, which precipitated upon addition of heptane, was recovered by filtration. A total yield of 3.65 grams of Compound 3 was recovered in 91 percent yield.

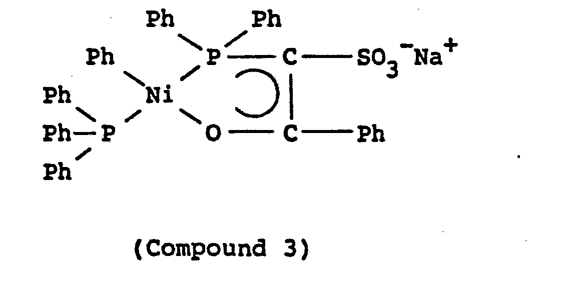

When Example I above was repeated except that Compound 1 was titrated with potassium hydroxide, ammonium hydroxide and trimethylphenylammonium hydroxide in place of 10 percent aqueous sodium hydroxide the following novel nickel ylides, respectively, were obtained: 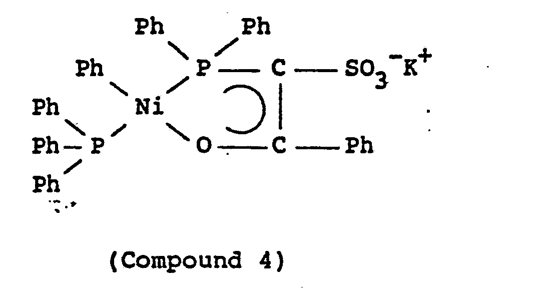 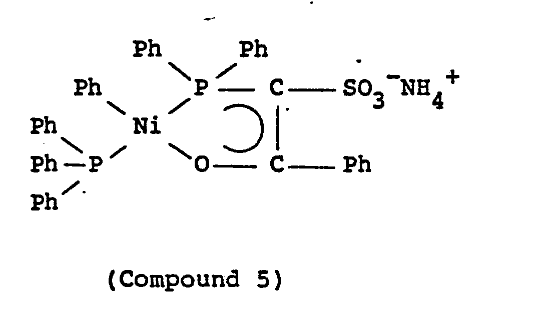 and

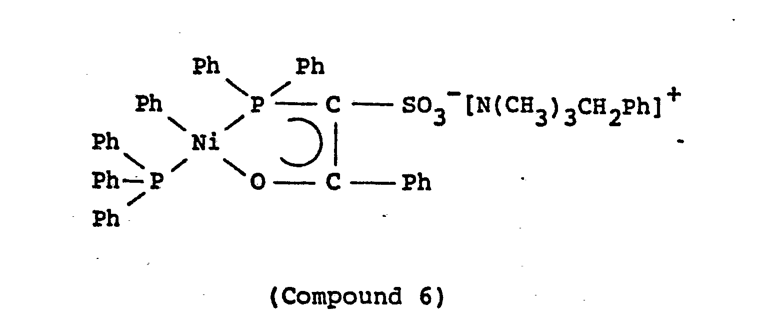

In producing Compounds 4, 5 and 6 identified above, it is apparent that the following ylides corresponding to Compound 2, respectively, will also be obtained: 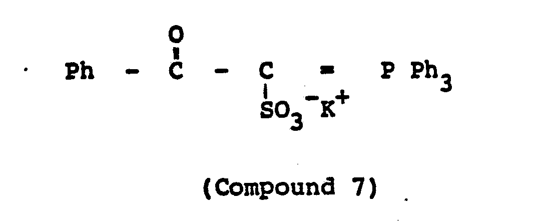 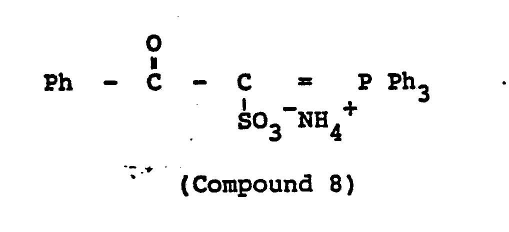 and

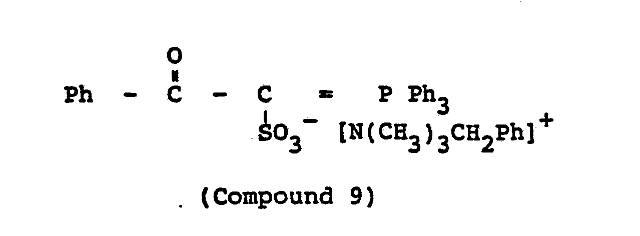

### Example II

A run was carried out wherein there was charged 0.1 millimole of the sulfonated nickel ylide catalyst obtained in Example I, Compound 3, dissolved in 100 milliliters of toluene. During the reaction precautions were taken to exclude air contamination by performing the reaction in an argon atmosphere. The reaction mixture was then heated to 50^{*}C. and pressured with ethylene to obtain a partial pressure thereof of 200 pounds per square inch gauge (1400 kPa). The reaction mixture was stirred throughout the reaction period of two hours, during which time the temperature and pressure were maintained constant. At the end of the two-hour period the reaction mixture was cooled to room temperature and unreacted ethylene removed therefrom by distillation. The amount of oligomer produced was determined and compared with the activity for the compound reported by the Keim et al article previously discussed. The results obtained are set forth in Table II.

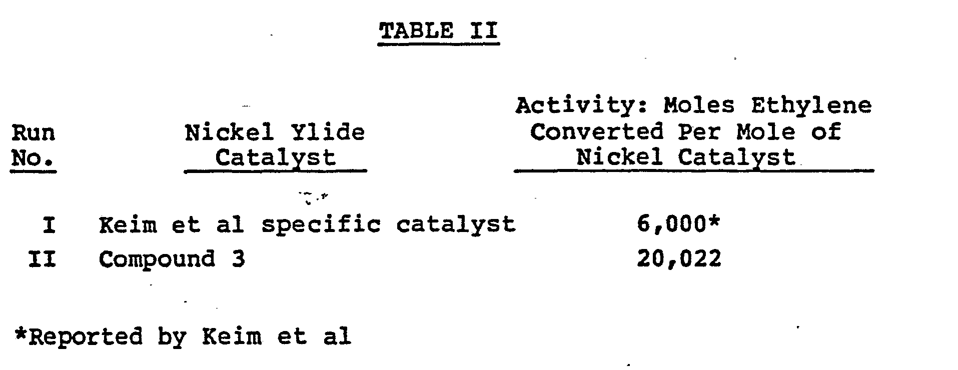

Compound 3 is more active than the unsulfonated nickel ylide of Keim et al. An additional advantage of Compound 3 over that of Keim et al lies in its easy recovery from the reaction product.

### Example III

An additional series of runs were carried out similar to Run No. II but wherein the reactions were carried out at specific elevated temperatures. These data are summarized below in Table III.

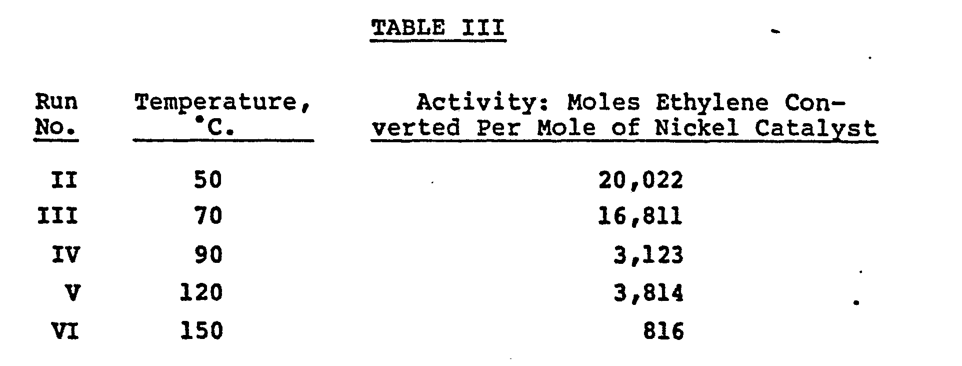

Although the invention has been described in considerable detail with particular reference to certain preferred embodiments thereof, variations and -modifications can be effected within the spirit and scope of the invention as described hereinbefore, and as defined in the appended claims.

## Claims

1. A process for preparing a nickel ylide having the general formula 1: 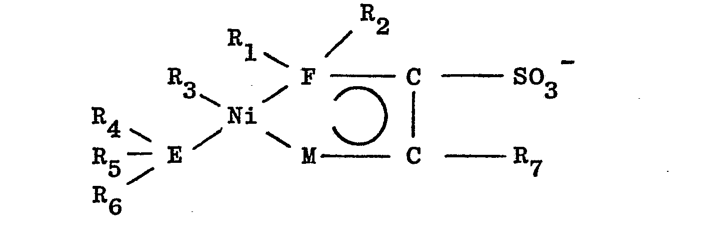 said process comprising sulfonating a metal ylide defined by the following formula: 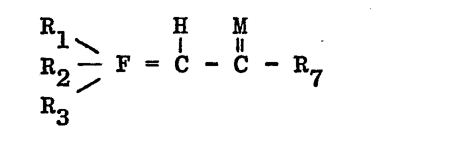 to obtain the sulfonated ylide defined by the following formula: 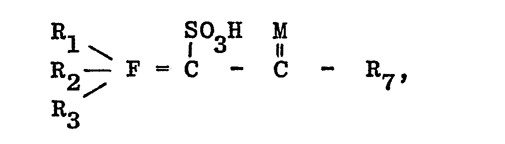 reacting the sulfonated ylide with a base to obtain the metal ylide defined by the following formula: 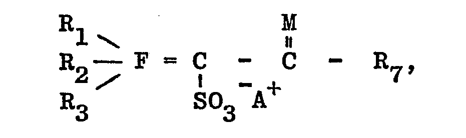 and then reacting the latter metal ylide with (1) a ligand defined by the following formula: 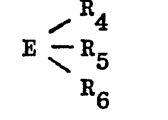 and (2) a zero valent nickel compound, in which formulae R₁, R_{2'} R₃ and R₇₁ alike or different, are selected from hydrogen; hydrocarbyl groups selected from alkyl radicals of from one to 24 carbon atoms; aryl radicals of from 6 to 20 carbon atoms, alkenyl radicals of from 2 to 30 carbon atoms, cycloalkyl radicals of from 3 to 40 carbon atoms, aralkyl and alkaryl raidcals of from 6 to 40 carbon atoms; halogen atoms selected from fluorine, chlorine, bromine and iodine; hydroxyl, alkoxy, aryloxy and said hydrocarbyl groups defined above having one or more substituents selected from halogen atoms, hydroxyl, alkoxy and aryloxy: R₄, R₅ and R₆₂ alike or different, are selected from said hydrocarbyl groups defined above, sulphonato groups and sulphonated hydrocarbyl groups: M is sulphur or oxygen: E is phosphorus, arsenic, antimony or nitrogen: F is phosphorus, arsenic or antimony, and A⁺ is the cationic portion of the base used.

2. A process as claimed in claim 1 wherein E and F are both phosphorus and M is oxygen.

3. A process as claimed in claim 1 or claim 2 wherein each of R₁, R_{2'} R₃, R₄, R₅₂ R₆ and R₇ is phenyl.

4. A process as claimed in any preceding claim wherein said sulfonation is carried out by dissolving said ylide in a solvent and then adding S0₃ to the resulting solution.

5. A process as claimed in claim 4 wherein said sulfonation is carried out at a temperature of from 0° to 200°C for from five minutes to 24 hours.

6. A process as claimed in claim 5 wherein said sulfonation is carried out at a temperature of from 20° to 100°C for from ten minutes to four hours.

7. A process as claimed in any preceding claim wherein said sulfonated ylide is reacted with said base at a temperature of from room temperature to 200°C for from one minute to four hours.

8. A process as claimed in claim 7 wherein said sulfonated ylide is reacted with said base at a temperature of from room temperature to 50°C for from one to two hours.

9. A process as claimed in any preceding claim wherein the ligand, the zero valent nickel compound and the ylide are reacted at a temperature of from 0° to 100°C for from 30 minutes to 48 hours.

10. A process as claimed in claim 9 wherein the ligand, the zero valent nickel compound and the ylide are reacted at room temperature for from three to 20 hours.

11. A process as claimed in any preceding claim wherein said zero valent nickel compound is bis(cyclooctadiene) nickel.

12. A process as claimed in any preceding claim wherein said base is selected from sodium hydroxide, potassium hydroxide, ammonium hydroxide and trimethylphenylammonium hydroxide.

13. A process for preparing a sulfonated ylide defined by the following formula: 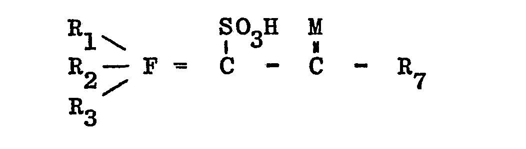 which comprises dissolving the ylide defined by the following formula: 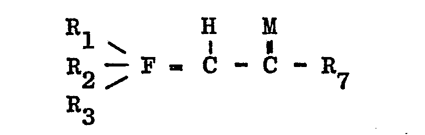 in a solvent and then adding SO₃ to the resulting solution, wherein R₁, R₂₁ R₃₁ R₄, R₇₁ F and M are as defined in claim 1.

14. A process as claimed in claim 13 wherein F is phosphorus and M is oxygen.

15. A process as claimed in claim 13 or claim 14 wherein each of R₁, R_{2'} R3 and R₇ is phenyl.

16. A process as claimed in claim 13 or 14 or 15 wherein said sulfonation is carried out at a temperature of from O° to 200°C for from five minutes to 24 hours.

17. A process as claimed in claim 16 wherein said sulfonation is carried out at a temperature of from 20° to 100°C for from ten minutes to four hours.

18. A process for preparing a ylide defined by the following formula: 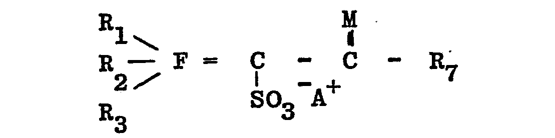 which comprises reacting a sulfonated ylide defined by the following formula: 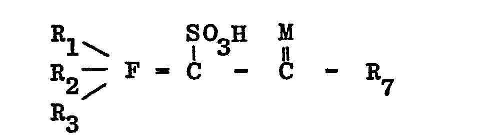 with a base wherein Rₗ, R₂₂ R₃, R₄, R₇, F, M and A+ are as defined in claim 1.

19. A process as claimed in claim 18 wherein F is phosphorus and M is oxygen.

20. A process as claimed in claim 18 or claim 19 wherein each of R₁, R₂ and R₃ is phenyl.

21. A process as claimed in claim 18 or 19 or 20 wherein said base is selected from sodium hydroxide, potassium hydroxide, ammonium hydroxide and trimethylphenylammonium hydroxide.

22. A process as claimed in any of claims 18 to 21 wherein said sulfonated ylide is reacted with said base at a temperature of from room temperature to 200°C for from one minute to four hours.

23. A process as claimed in claim 22 wherein said sulfonated ylide is reacted with said base at a temperature of from room temperature to 50°C for from one to two hours.

24. A metal ylide defined by the following formula: 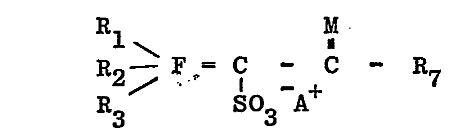 wherein Rₗ, R₂, R₃, R₇, F, M and A⁺ are as defined in claim 1.

25. A metal ylide as claimed in claim 24 wherein F is phosphorus and M is oxygen.

26. A metal ylide as defined in claim 24 or claim 25 wherein each of R₁, R₂₁ ^{R}₃ and R₇ is phenyl.

27. A metal ylide as claimed in claim 24 or 25 or 26 wherein A is sodium or potassium or trimethylphenylammonium.
